# EUROPÄISCHE PATENTSCHRIFT

(11) **EP 0 668 271 B1**
(45) Veröffentlichungstag und Bekanntmachung des Hinweises auf die Patenterteilung: **16.01.2002**
(21) Anmeldenummer: 95101740.9
(22) Anmeldetag: 09.02.1995
(51) Int. Cl.: C07D 251/34, C08G 18/79

(54) **Verfahren zur Herstellung von Isocyanuratgruppen aufweisenden Polyisocyanaten**
Process for the preparation of isocyanurate-group containing polyisocyanates
Procédé pour la préparation de polyisocyanates contenant des groupes isocyanurates

(30) Priorität: 17.02.1994 DE 4405055
(43) Veröffentlichungstag der Anmeldung: 23.08.1995
(73) Patentinhaber: BASF Aktiengesellschaft, 67063 Ludwigshafen (DE)
(72) Erfinder: Brandt, Dr. Peter, D-67059 Ludwigshafen (DE); Bruchmann, Dr. Bernd, D-67069 Ludwigshafen (DE); Laqua, Dr. Gerhard, D-68167 Mannheim (DE); Merger, Dr. Franz, D-67227 Frankenthal (DE); Otterbach, Dr. Andreas, D-67227 Frankenthal (DE); Stiefenhöfer, Konrad, D-67280 Ebertsheim (DE); Witzel, Dr. Tom, D-67069 Ludwigshafen (DE); Wolff, Dr. Stefan, D-67117 Limburgerhof (DE); Becker, Dr. Rainer, D-67098 Bad Dürkheim (DE)

(56) Entgegenhaltungen:
- EP-A- 0 512 213
- DE-A- 2 631 733
- US-A- 3 703 520
- US-A- 4 454 317

## Beschreibung

Die vorliegende Erfindung betrifft ein neues Verfahren zur Herstellung von Isocyanuratgruppen aufweisenden Polyisocyanaten durch eine partielle Trimerisierung von (cyclo)aliphatischen Diisocyanaten, vorzugsweise von nach phosgenfreien Verfahren erhältlichen (cyclo)aliphatischen Diisocyanaten, in Gegenwart mindestens eines Trimerisierungskatalysators aus der Gruppe der Tetraalkylammoniumalkylcarbonate und betainstrukturierten (Quartär-Ammonioalkyl)-carbonate und die bevorzugte Verwendung der so erhältlichen Isocyanuratgruppen aufweisenden Polyisocyanate als Polyisocyanatkomponente in Polyurethanlacken.

Verfahren zur partiellen oder vollständigen Trimerisierung von organischen Polyisocyanaten zur Herstellung von Isocyanuratgruppen aufweisenden Polyisocyanaten oder zelligen oder kompakten Isocyanuratgruppen aufweisenden Polyurethanen sind bekannt und werden in zahlreichen Literaturveröffentlichungen, wie z.B. dem Kunststoff-Handbuch, Polyurethane, Band 7, 2. Auflage 1983 (Seite 81) herausgegeben von Dr. G. Oertel, Carl Hanser Verlag, München, Wien, in Polyurethanes, Chemistry and Technology, Part I, 1962, von J.H. Saunders and K.C. Frisch, (Seite 94), Advances in Urethane Science and Technology, Vol. 3, (Technomic Publishing Co., Inc. 1974, Seite 141) und Plaste und Kautschuk 23, Seiten 162ff und 177ff (1976), und Patentschriften beschrieben. Als geeignete Trimerisierungskatalysatoren werden beispielsweise Alkalioxide, Alkalihydroxide und starke organischen Basen, wie z.B. Alkalialkoholate, -phenolate, Metallsalze von Carbonsäuren, beispielsweise Kobaltnaphthenat, Natriumbenzoat, Natriumacetat und Kaliumformiat, tertiäre Amine, beispielsweise Triethylamin, N,N-Dimethylbenzylamin, Triethylendiamin, Tris-2,4,6-(dimethylaminomethyl)-phenol und Tris-1,3,5-(dimethylaminopropyl)-S-hexahydrotriazin, tertiäre Phosphine und tertiäre Ammoniumverbindungen genannt.

Nach Angaben der DE-A-38 06 276 haben sich als Trimerisierungskatalysatoren quarternäre Ammoniumhydroxide, vorzugsweise N,N,N-Trimethyl-N-benzylammoniumhydroxid und N,N,N-Trimethyl-N-(2-hydroxypropyl)-ammoniumhydroxid bewährt. Nachteilig an diesen Katalysatoren ist, daß sie sehr empfindlich gegenüber Verunreinigungen sind und die zur Trimerisierung verwendbaren Diisocyanate von Kohlendioxid bis auf einen Restgehalt von weniger als 20 ppm befreit werden müssen. Die EP-A-0 339 396 (US-A-4 960 848) beschreibt als Trimerisierungskatalysator quarternäre Ammonium-fluoride und die EP-A-0 379 914 (US-A-5 013 838) nennt als Katalysatoren quarternäre Ammonium- oder Phosphoniumfluoride, wobei die Trimerisierung in Gegenwart von in das Reaktionsgemisch eingeleitetem Kohlendioxid durchgeführt wird. Nachteilig an den genannten Trimerisierungskatalysatoren ist, daß sie bei der Umsetzung von Hexamethylen-diisocyanat, abgekürzt auch HDI genannt, überwiegend trübe, für die Lackanwendung unbrauchbare Produkte ergeben. Trübungsfreie Isocyanuratgruppen enthaltende Polyisocyanate werden, nach Angaben der EP-A-0 010 589 (US-A-4 324 879) bei Verwendung von Hydroxyalkyl substituierten quarternären Ammoniumhydroxiden als Katalysator erhalten. Nachteilig bei diesem Verfahren ist, daß sich die Hydroxyalkylammoniumhydroxide nur sehr schwierig farblos herstellen lassen und in verhältnismäßig großen Mengen, beispielsweise in Mengen bis zu 0,6 Gew.-%, angewendet werden müssen. Die von überschüssigem HDI befreiten Isocyanuratgruppen aufweisenden Polyisocyanate können daher eine gelbliche Färbung aufweisen. Zur Trimerisierung und Polymerisation von Polyisocyanaten und zur Polyaddition von Polyisocyanaten und Polyolen werden in der US-A-3 817 939 als Katalysatoren organische Metallsalze der Formel (A)ₙ-R-O-C^{⊖}O^{⊕} beschrieben, in der bedeuten, A eine Hydroxylgruppe oder ein Wasserstoffatom, n eine Zahl von 1 bis 3, R einen polyfunktionellen linearen oder verzweigten, aliphatischen oder aromatischen Kohlenwasserstoffrest und M ein Kation einer starken Base, z.B. ein Alkalimetallkation oder ein quarternäres Ammoniumkation, wie Tetraalkylammonium. Die US-A-3 703 520 nennt als Trimerisierungskatalysatoren Addukte aus Ethylencarbonat und Triethylendiamin in im wesentlichen äquimolaren Verhältnissen. Nachteilig sind auch bei diesen Verfahren die zur Trimerisierung erforderlichen hohen Katalysatormengen von 0,5 bis 5 Gew.-%, bezogen auf die Polyiso-cyanatmenge. Die DE-A-26 31 733 (US-A-4 040 992) beschreibt ein Verfahren zur Herstellung von Polyurethanen und Polyisocyanuraten aus organischen Polyisocyanaten und gegebenenfalls Polyolen in Gegenwart von quartären Hydroxyalkylammoniumverbindungen der Formel 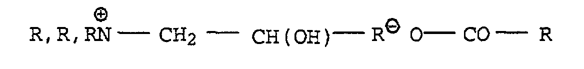 als Katalysator. Nachteilig an diesen Katalysatoren ist ihre thermische Labilität, so daß sie nur in einem engen Temperaturbereich einsetzbar sind, und ihre geringe katalytische Wirksamkeit, die zu Verfärbungen des Endprodukts führen kann.

In EP-A-0 512 213 wird ein Verfahren zur Herstellung von blockierten Lochpolyisocyanaten durch Trimerisierung von Isophorondiisocyanat mit quarternären N-(Hydroxyl)-ammoniumsalzen und anschließende Blockierung der Isocyanatgruppen mit ε-Caprolactam. Nachteilig ist auch hier die geringe thermische Stabilität der eingesetzten Katalysatoren.

US-A-4,454,317 beschreibt ebenfalls die Trimerisierung von Isocyanaten mit quarternären Ammoniumsalzen. Auch hier ist die thermische Stabilität der Katalysatoren gering.

Bekannt sind ferner die Herstellung von quarternären Ammoniumcarbonaten durch Umsetzung von tertiären Aminen und Dialkylcarbonaten in einem bevorzugten Molverhältnis von 2 : 1 bis 3 : 1 gemäß US-A-2 635 100 und betainartige Kohlensäurehalbester der Formel 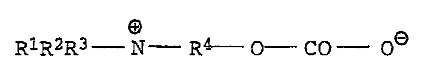 sowie ein Verfahren zu ihrer Herstellung aus der EP-A-0 400 435. In den genannten Patentpublikationen nicht beschrieben wird die Verwendung der quarternären Ammoniumcarbonate und betainartigen Kohlensäurehalbester zur Herstellung von Isocyanuratgruppen enthaltenden Polyisocyanaten.

Die Aufgabe der vorliegenden Erfindung bestand darin, im wesentlichen farblose Isocyanuratgruppen enthaltende Polyisocyanate, die als Polyisocyanatkomponente für vorzugsweise Polyurethan (PU) - Lacksysteme verwendbar sind, nach einem möglichst einfachen Verfahren in sehr guter Qualität reproduzierbar, vorzugsweise aus nach phosgenfreien Verfahren erhältlichen (cyclo)aliphatischen Diisocyanaten, herzustellen.

Diese Aufgabe konnte überraschenderweise gelöst werden durch die Verwendung von Tetraalkylammoniumalkylcarbonaten oder/und betainstrukturierten (Quartär-Ammonioalkyl)carbonaten als Trimerisierungskatalysator.

Gegenstände der Erfindung sind somit ein Verfahren zur Herstellung von Isocyanuratgruppen aufweisenden Polyisocyanaten durch partielle Trimerisierung von aliphatischen oder/und cycloaliphatischen Diisocyanaten in Gegenwart mindestens eines Trimerisierungskatalysators und Desaktivierung des Trimerisierungskatalysators nach Erreichen des angestrebten Trimerisierungsgrads, das dadurch gekennzeichnet ist, daß man als Trimerisierungskatalysatoren Tetraalkylammoniumalkylcarbonate der Formel (I) 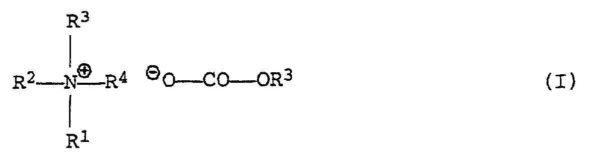 oder betainstrukturierte (Quartar-Ammonioalkyl) carbonate der Formel (II) 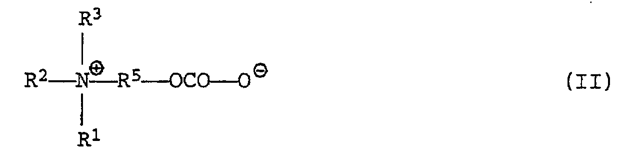 oder Mischungen aus (I) und (II) verwendet, in denen
R¹, R² und R⁴ gleich oder verschieden sind und für eine C₁- bis C₂₀-Alkylgruppe, vorzugsweise eine C₁- bis C₆-Alkylgruppe, eine C₅- oder C₆-Cycloalkylgruppe, eine C₇- bis C₁₀-Aralkylgruppe, vorzugsweise C₇-Aralkylgruppe oder eine Phenylgruppe stehen oder in denen R¹ und R² zusammen mit dem Stickstoffatom einen 5- oder 6-gliedrigen Ring bilden, der noch ein zusätzliches Stickstoff- oder Sauerstoffatom als Brückenglied enthalten kann oder in denen R¹, R² und R⁴ gemeinsam mit dem Stickstoffatom ein mehrgliedriges, vorzugsweise sechsgliedriges Mehrringsystem, vorzugsweise Zwei ringsystem bilden, das noch ein oder mehrere zusätzliche Stickstoffatome, Sauerstoffatome oder Stickstoff- und Sauerstoffatome als Brückenglieder enthalten kann,
R³ eine C₁- bis C₄-Alkylgruppe, vorzugsweise eine Methylgruppe ist,
R⁵ eine C₂- bis C₂₀-Alkylengruppe, vorzugsweise eine C₂- bis C₁₂-Alkylengruppe, eine C₅- oder C₆-Cycloalkylengruppe, eine C₇- bis C₁₀-Aralkylengruppe, vorzugsweise Benzylengruppe oder eine Phenylengruppe bedeuten oder
R⁵ und R¹ zusammen eine Alkylengruppe sind, die gemeinsam mit einem Stickstoffatom als Brückenglied einen 5- bis 7-gliedrigen, vorzugsweise 6-gliedrigen heterocyclischen Ring bilden.

Die erfindungsgemäß als Trimerisierungskatalysatoren verwendbaren Tetraalkylammoniumalkylcarbonate und betainstrukturierte (Quartär-Ammonioalkyl)carbonate sind bei Temperaturen über 100°C thermisch beständig und somit über einen Temperaturbereich von ungefähr 20 bis 180°C katalytisch wirksam, während die quartären Hydroxyalkylammoniumcarboxylate nach DE-A-26 31 733 aufgrund ihrer thermischen Labilität in der Regel nur bei Temperaturen von 60 bis 80°C verwendbar sind. Höhere Trimerisierungstemperaturen, beispielsweise über 95°C, werden zweckmäßigerweise zur Trimerisierung von sterisch behinderten Diisocyanaten, wie z.B. Iso-phorondiisocyanat oder 2-Butyl-2-ethyl-pentandiisocyanat-1,5 angewandt, da dadurch höhere Raum-Zeit-Ausbeuten erreicht werden können. Die Reaktionsgeschwindigkeit der Trimerisierungsreaktion kann bei Verwendung der erfindungsgemäßen Tetraalkylammoniumalkylcarbonate um den Faktor 2 bis 8 und bei Verwendung von betainstrukturierten (Quartär-Ammonioalkyl)-carbonaten um den Faktor 15 bis 20 erhöht werden oder es kann die eingesetzte Menge der erfindungsgemäßen Katalysatoren im Vergleich zu z.B. N-(2-Hydroxypropyl-N,N,N-trimethylammoniummono-2-ethylhexanoat beträchtlich verringert werden.

Die erfindungsgemäßen neuen Trimerisierungskatalysatoren können zur Trimerisierung von nach beliebigen Verfahren hergestellten (cyclo)aliphatischen Diisocyanaten verwendet werden. Besonders bewährt haben sie sich jedoch und daher vorzugsweise verwendet werden sie zur Trimerisierung von nach phosgenfreien Verfahren hergestellten (cyclo)aliphatischen Diisocyanaten, da hierbei nicht nur die Reaktionsgeschwindigkeit der Trimerisierungsreaktion beträchtlich erhöht werden kann, sondern auch Isocyanuratgruppen aufweisende Polyisocyanate mit äußerst niedrigen Farbzahlen nach Hazen, z.B. vorzugsweise kleiner als 35 erhalten werden.

Wie bereits dargelegt wurde, können die erfindungsgemäß verwendbaren Trimerisierungskatalysatoren nach bekannten Verfahren hergestellt werden. Zur Herstellung von Tetraalkylammoniumalkylcarbonaten der Formel (I), vorzugsweise von Trialkylmethylammoniummethylcarbonat, können z.B. Dialkylcarbonate, vorzugsweise Dimethylcarbonat, mit tertiären Aminen in Abwesenheit oder Gegenwart von Lösungsmitteln, z.B. Chlorbenzol, bei Temperaturen von zweckmäßigerweise 100 bis 180°C zur Reaktion gebracht werden. Als geeignete tertiäre Amine seien beispielhaft genannt: N,N'-Dimethyl-piperazin, N-Methoxyphenyl-piperazin, N-Methylpiperidin, N-Ethyl-piperidin, Chinuclidin, Trialkylamine, wie z.B. Trimethyl-, Triethyl- und Tripropylamin, und vorzugsweise 1,4-Dimethyl-piperazin, N,N-Dimethylbenzylamin und Triethylendiamin.

Die vorzugsweise als Trimerisierungskatalysatoren verwendeten Tetraalkylammoniumalkylcarbonate, die ebenso wie andere geeignete Tetraalkylammoniumalkylcarbonate einzeln oder in Form von Mischungen verwendet werden können, weisen die Formeln (III), (IV) und (VII) auf 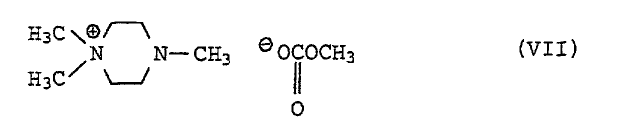 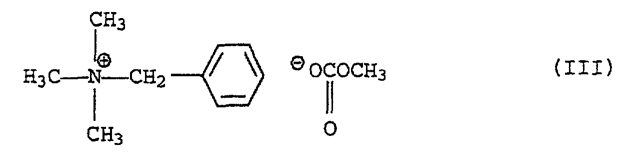 und 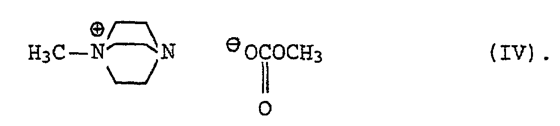 Die Herstellung der betainstrukturierten (Quartär-Ammonioalkyl)-carbonate der Formel (II) kann z.B. durch Umsetzung von Dialkylcarbonaten, vorzugsweise Dimethylcarbonat, mit Hydroxylgruppen aufweisenden tertiären Aminen bei Temperaturen von 25 bis 200°C entsprechend den Angaben der EP-A-400 435 durchgeführt werden.

Als geeignete hydroxylgruppenhaltige tertiäre Amine seien beispielsweise genannt: N,N-Dialkylaminoalkanole, wie z.B. N,N-Dimethylaminoethanol, N,N-Dimethylaminobutanol, N,N-Dimethylaminooctanol, N,N-Diethylaminoethanol, N,N-Diethylaminopropanol, N,N-Diethylaminobutanol, N,N-Diethylaminopentanol, N,N-Diisopropylaminoethanol, N-Butyl-N-methylaminopropanol, N-Cyclohexyl-N-methylaminoethanol, N-Benzyl-N-propylaminoethanol, N-Hydroxyethyl-pyrrolidin, N-Hydroxyethylpiperidin, N-Hydroxyethylmorpholin, N-Hydroxypropylpyrrolidin, N-Hydroxypropylpiperidin, N-Hydroxypropylmorpholin, 3-Hydroxychinuclidin, N,N-Dimethylaminoneopentanol, 1-Hydroxymethyl-1-dimethylaminomethylcyclohexan, N,N-Diethylaminoneopentanol, N,N-Dibutylaminoneopentanol, Pyrrolidinoneopentanol, Piperidinoneopentanol, 3-Hydroxymethyl-3-dimethyl-aminomethyl-heptan, 3-Hydroxymethyl-3-diethylaminomethylheptan, 3-Hydroxymethyl-3-(N-methyl-N-hexylamino)-heptan, (N-Methoxyethyl-N-methylamino)-ethanol, (N-Butoxyethyl-N-ethylamino)ethanol, (N-Phenoxyethyl-N-methylamino)-propanol, N-Methyl-3-pyrrolidinol, und vorzugsweise N,N-Dimethylaminopropanol, N,N-Dimethylaminohexanol und N-Methyl-4-piperidinol.

Die vorzugsweise als Trimerisierungskatalysatoren verwendeten betainstrukturierten (Quartär-Ammonioalkyl)-carbonate, die ebenso wie andere geeignete betainstrukturierten (Quartär-Ammonioalkyl)-carbonate einzeln oder in Form von Mischungen verwendet werden können, weisen die Formeln (V), (VI) und (VIII) auf 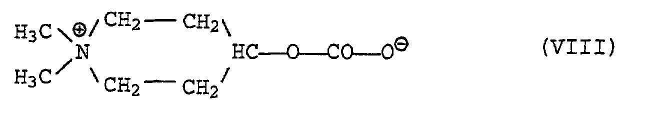 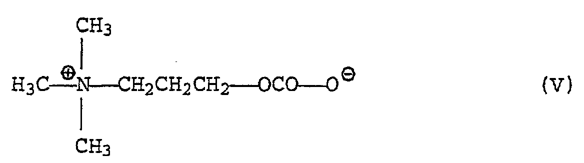 und

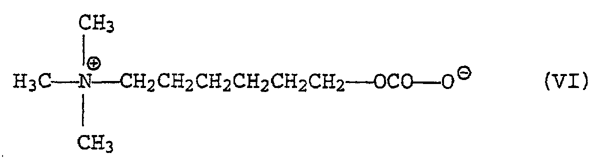

Als Trimerisierungskatalysatoren können auch Gemische aus Tetraalkylammoniumalkylcarbonaten (I) und Tetraalkylammoniumcarbonaten mit Betainstruktur (II) verwendet werden, wobei diese in breiten Mengenverhältnissen, z.B. in Mengenverhältnissen von 90 : 10 bis 10 : 90 und vorzugsweise 60 : 40 bis 40 : 60 gemischt werden können.

Zur Herstellung der Isocyanuratgruppen aufweisenden Polyisocyanate werden die erfindungsgemäßen Trimerisierungskatalysatoren in Abhängigkeit von ihrer katalytischen Wirksamkeit zweckmäßigerweise in möglichst kleinen wirksamen Mengen, die experimentell auf einfache Weise ermittelt werden können, verwendet.

Im allgemeinen gelangen beim erfindungsgemäßen Verfahren die Tetraalkylammoniumalkylcarbonate (I) in einer Menge von 0,002 bis 0,05 Gew.-%, vorzugsweise von 0,005 bis 0,02 Gew.-% und die betainstrukturierten (Quartär-Ammonioalkyl)-carbonate (II) in einer Menge von 0,001 bis 0,03 Gew.-%, vorzugsweise von 0,002 bis 0,01 Gew.-%, jeweils bezogen auf das Gewicht der (cyclo)aliphatischen Diisocyanate, zum Einsatz.

Das erfindungsgemäße Verfahren wird zweckmäßigerweise bei einer Temperatur im Bereich von 30 bis 150°C durchgeführt. Bei Temperaturen unter 30°C kann eine Gelbildung eintreten, die auf eine lineare Polymerisation der (cyclo)aliphatischen Diisocyanate zu Nylon-1-Strukturen zurückzuführen ist. Bei Temperaturen über 150°C kann eine Verfärbung der Isocyanuratgruppen aufweisenden Polyisocyanate auftreten, z.B. bei längeren Reaktionszeiten.

Bei Verwendung von Tetraalkylammoniumalkylcarbonaten (I) werden vorzugsweise Reaktionstemperaturen über 80°C, insbesondere von 85 bis 120°C angewandt, während bei Verwendung von betainstrukturierten (Quartär-Ammonioalkyl)-carbonaten (II) Reaktionstemperaturen unterhalb 80°C bevorzugt sind, wobei insbesondere bei Reaktionstemperaturen von 60 bis 40°C im wesentlichen farblose Trimerisierungsprodukte erhalten werden.

Nach Erreichen des gewünschten Trimerisierungsgrads bzw. NCO-Gehalts der Isocyanurat/(cyclo)aliphatische Diisocyanat-Reaktionsmischung, der zweckmäßigerweise im Bereich von 45 bis 25 Gew.-% NCO-Gruppen, vorzugsweise von 43 bis 38 Gew.-% NCO-Gruppen, liegt und wozu üblicherweise Reaktionszeiten von 0,05 bis 4 Stunden, vorzugsweise von 1 bis 2 Stunden, erforderlich sind, wird die Trimerisierungsreaktion durch eine Desaktivierung des Trimerisierungskatalysators beendet. Als Desaktivierungsmittel eignen sich beispielsweise anorganische Säuren, wie z.B. Salzsäure, phosphorige Säure oder Phosphorsäure, Carbonsäurehalogenide wie z.B. Acetylchlorid oder Benzoylchlorid, Sulfonsäuren oder -ester, wie z.B. Methansulfonsäure, p-Toluolsulfonsäure, p-Toluolsulfonsäuremethyl- oder -ethylester, m-Chlorperbenzoesäure und vorzugsweise Dialkylphosphate wie z.B. Di-2-ethylhexylphosphat und insbesondere Dibutylphosphat. Die Desaktivierungsmittel können, bezogen auf die Trimerisierungskatalysatoren, in äquivalenten, überschüssigen oder unterschüssigen Mengen verwendet werden, wobei die kleinste wirksame Menge, die experimentell ermittelt werden kann, schon aus wirtschaftlichen Gründen bevorzugt wird.

Das erfindungsgemäße Verfahren wird vorzugsweise lösungsmittelfrei durchgeführt. Werden die (cyclo)aliphatischen Diisocyanate jedoch in Gegenwart von Lösungs- oder Verdünnungsmitteln partiell trimerisiert, so eignen sich hierfür sowohl inerte unpolare als auch inerte polare Lösungs- oder Verdünnungsmittel, wie z.B. Toluol, Xylol, cyclische Ether, Carbonsäureester und Ketone oder ihre Gemische.

Die nach dem erfindungsgemäßen Verfahren hergestellten Isocyanuratgruppen aufweisende Polyisocyanate können in an sich bekannter Weise, beispielsweise durch Dünnschichtdestillation oder Extraktion, von gegebenenfalls vorhandenen Lösungs- oder Verdünnungsmittel und/oder vorzugsweise von überschüssigen, nicht-umgesetzten (cyclo)aliphatischen Diisocyanaten befreit werden, so daß die Isocyanuratgruppen aufweisenden Polyisocyanate mit einem Gehalt an monomeren Diisocyanaten von z.B. unter 1,0 Gew.-%, vorzugsweise unter 0,5 Gew.-% erhältlich sind.

Die Abtrennung der überschüssigen monomeren Diisocyanate erfolgt zweckmäßigerweise bei Verwendung der Verfahrensprodukte zur Herstellung von Polyurethanlacken. Ohne Abtrennung der überschüssigen monomeren Diisocyanate eignen sich die Isocyanuratgruppen aufweisende Polyisocyanate beispielsweise zur Herstellung von PU-Schaumstoffen, zelligen oder kompakten Elastomeren, Vergußmassen und Klebstoffen. Die monomerfreien und monomerhaltigen Isocyanuratgruppen aufweisenden Polyisocyanaten können ferner in an sich bekannter Weise durch Einführung von z.B. Urethan-, Allophanat-, Harnstoff-, Biuret- und/oder Carbodiimidgruppen modifiziert und/oder die Isocyanatgruppen mit geeigneten Blockierungsmitteln, wie z.B. ε-Caprolactam, Malonsäuredimethylester, Acetessigester oder aromatischen Hydroxylgruppen blockiert werden.

Nach dem erfindungsgemäßen Verfahren können beliebige, organische Diisocyanate mit aliphatischen, cycloaliphatischen oder aliphatischen und cycloaliphatischen Isocyanatgruppen oder ihre Gemische trimerisiert werden.

Geeignete aliphatische Diisocyanate besitzen vorteilhafterweise 3 bis 16 C-Atome, vorzugsweise 4 bis 12 C-Atome im linearen oder verzweigtkettigen Alkylenrest und geeignete cycloaliphatische Diisocyanate vorteilhafterweise 4 bis 18 C-Atome, vorzugsweise 6 bis 15 C-Atome im Cycloalkylenrest. Beispielhaft genannt seien: 1,4-Diisocyanato-butan, 2-Ethyl-1,4-diisocyanato-butan, 1,5-Diisocyanato-pentan, 2-Methyl-1,5-diisocyanato-pentan, 2,2-Dimethyl-1,5-diisocyanato-pentan, 2-Propyl-2-ethyl-1,5-diisocyanato-pentan, 2-Butyl-2-ethyl-1,5-diisocyanato-pentan, 2-Alkoxymethylen-1,5-diisocyanato-pentan, 3-Methyl-, 3-Ethyl-1,5-diisocyanato-pentan, 1,6-Hexamethylen-diisocyanat, 2,4,4- bzw. 2,2,4-Trimethyl-1,6-hexamethylen-diisocyanat, 1,7-Diisocyanatoheptan, 1,8-Diisocyanato-octan, 1,10-Diisocyanato-decan, 1,12-Diiso-cyanato-dodecan, 4,4'-Diisocyanato-dicyclohexylmethan, 2,4'-Diisocyanato-dicyclohexylmethan sowie Gemische der Diisocyanato-dicyclohexylmethan-Isomeren, 1,3-Diisocyanato-cyclohexan sowie Isomerengemische von Diisocyanato-cyclohexanen und 1-Isocyanato-3-isocyanatomethyl-3,5,5-trimethylcyclohexan. Als (cyclo)aliphatische Diisocyanate vorzugsweise verwendet werden 1,6-Hexamethylen-diisocyanat, isomere aliphatische Diisocyanate mit 6 Kohlenstoffatomen im Alkylenrest sowie Mischungen davon, 2-Butyl-2-ethyl-1,5-diisocyanato-pentan und 1-Isocyanato-3-isocyanatomethyl-3,5,5-trimethylcyclohexan.

Die erfindungsgemäß verwendbaren (cyclo)aliphatischen Diisocyanate können nach beliebigen Verfahren, beispielsweise durch Phosgenierung der entsprechenden Diamine und thermische Spaltung der intermediär gebildeten Dicarbaminsäurechloride hergestellt werden. Nach phosgenfreien Verfahren hergestellte (cyclo)aliphatische Diisocyanate enthalten als Nebenprodukte keine Chlorverbindungen und besitzen daher ein grundsätzlich anderes Nebenproduktspektrum. Überraschenderweise zeigte sich, daß die erfindungsgemäß verwendbaren Trimerisierungskatalysatoren bei der Trimerisierung von nach phosgenfreien Verfahren hergestellten (cyclo)aliphatischen Diisocyanaten eine deutlich höhere katalytische Wirksamkeit aufweisen und Isocyanuratgruppen aufweisende Polyisocyanate mit beträchtlich niedrigerer Farbzahl ergeben als bei analoger Reaktion von durch Phosgenierung hergestellten (cyclo)aliphatischen Diisocyanaten. Es hat sich daher als zweckmäßig erwiesen, nach dem erfindungsgemäßen Verfahren vorzugsweise nach einem phosgenfreien Verfahren und insbesondere durch thermische Spaltung von (cyclo)aliphatischen Dicarbaminsäureestern erhältliche (cyclo)aliphatische Diisocyanate zu verwenden, wobei durch thermische Spaltung von (cyclo)aliphatischen Dicarbaminsäureestern erhältliche Diisocyanate aus der Gruppe 1,6-Hexamethylen-diisocyanat, 2-Butyl-2-ethyl-pentamethylen-diisocyanat-1,5 und 1-Isocyanato-3-isocyanatomethyl-3,5,5-trimethylcyclohexan insbesondere bevorzugt verwendet werden. Nach diesen Verfahrensvarianten hergestellte Isocyanuratgruppen aufweisende Polyisocyanate eignen sich vorzüglich zur Herstellung von Polyurethan-Beschichtungen, z.B. von Textil- und Lederbeschichtungen, für -Dispersionen und -Klebstoffe und finden insbesondere Verwendung als Polyisocyanatkomponente in Zweikomponenten-Polyurethansystemen für hochwertige, wetterbeständige Polyurethan-Lacke und high-solid-Lacke.

### Beispiele

### Katalysatorherstellung

### Herstellung von Tetraalkylammoniumalkylcarbonaten der Formel I

1. Herstellung von 1,1-Dimethyl-1-azonia-4-methyl-4-aza[2,2]hexan-monomethylcarbonat der Formel (VII), im folgenden abgekürzt genannt: Kat. 1
In einem Reaktionsgefäß, ausgestattet mit Rührer, Rückflußkühler und Gasein- und -ableitungsrohr wurde eine Mischung aus 57,1 Gew.-Teilen (0,5 Mol) N,N'-Dimethylpiperazin und 90,1 Gew.-Teilen (1 Mol) Dimethylcarbonat unter Rühren in einer Inertgasatmosphäre (Stickstoff) auf 95°C erwärmt und bei dieser Temperatur 8 Stunden umgesetzt. Danach wurde das überschüssige Dimethylcarbonat abdestilliert und der erhaltene Destillationsrückstand mit 2500 Gew.-Teilen Methylethylketon aufgenommen. Das aus dem Methylethylketon auskristallisierte 1,1-Dimethyl-1-azonia-4-methyl-4-aza[2,2]hexan-monomethylcarbonat wurde unter einer Inertgasatmosphäre abgesaugt und getrocknet.

| Elementaranalyse (C₉H₂₀N₂O₃) | | | | |
|---|---|---|---|---|
| berechnet [Gew.-%] | C 52,9; | H 9,9; | N 13,7; | O 23,5 |
| gefunden [Gew.-%] | C 52,7; | H 9,1; | N 14,2; | O 24,3 |

Zersetzungsbereich: 220 bis 240°C
2. Herstellung von N,N,N-Trimethylbenzylammoniummonomethylcarbonat der Formel (III), im folgenden abgekürzt genannt: Kat. 2
Man verfuhr analog den Angaben zur Herstellung von Kat. 1, verwendete jedoch anstelle von N,N'-Dimethylpiperazin 67,6 Gew.-Teile (0,5 Mol) N,N-Dimethylbenzylamin

| Elementaranalyse (C₁₀H₁₉NO₃) | | | | |
|---|---|---|---|---|
| berechnet [Gew.-%] | C 63,9; | H 8,5; | N 6,2; | O 21,3 |
| gefunden [Gew.-%] | C 63,7; | H 8,6; | N 6,0; | O 21,6 |

Zersetzungsbereich: 210 bis 230°C
3. Herstellung von 1-Methyl-1-azonia-4-aza-[2,2,2]octanmonomethylcarbonat der Formel (IV), im folgenden abgekürzt genannt: Kat. 3
Man verfuhr analog den Angaben zur Herstellung von Kat. 1, verwendete jedoch anstelle von N,N'-Dimethylpiperazin 56,1 Gew.-Teile (0,5 Mol) Diazabicyclo-[2,2,2]-octan

| Elementaranalyse (C₉H₁₈N₂O₃) | | | | |
|---|---|---|---|---|
| berechnet [Gew.-%] | C 51,3; | H 8,1; | N 15,0; | O 25,6 |
| gefunden [Gew.-%] | C 51,3; | H 8,6; | N 14,3; | O 25,3 |

Zersetzungsbereich: 220 bis 240°C Herstellung von betainstrukturierten (Quartär-Ammonioalkyl) carbonaten der Formel (II)
4. Herstellung von 4-(N,N-Dimethylpiperidino)-carbonat der Formel (VIII), im folgenden abgekürzt genannt: Kat. 4
In einem Reaktionsgefäß, ausgestattet mit Rührer, Rückflußkühler und Gasein- und -ableitungsrohr wurde eine Mischung aus 73,6 Gew.-Teilen (0,5 Mol) N-Methyl-4-piperidinol und 90,1 Gew.-Teilen (1,0 Mol) Dimethylcarbonat unter Rühren in einer Inertgasatmosphäre (Stickstoff) auf 95°C erwärmt und bei dieser Temperatur 8 Stunden lang zur Reaktion gebracht. Danach wurde das überschüssige Dimethylcarbonat abdestilliert und der erhaltene Destillationsrückstand mit 2000 Gew.-Teilen Methylethylketon aufgenommen. Das aus dem Methylethylketon auskristallisierte 4-(N,N-Dimethylpiperidino)-carbonat wurde unter einer Inertgasatmosphäre abgesaugt und getrocknet.

| Elementaranalyse (C₈H₁₅NO₃) | | | | |
|---|---|---|---|---|
| berechnet [Gew.-%] | C 55,5; | H 8,7; | N 8,1; | O 27,7 |
| gefunden [Gew.-%] | C 55,3; | H 8,9; | N 8,3; | O 27,5 |

Zersetzungsbereich: 240 bis 260°C
5. Herstellung von N,N,N-Trimethylammoniumpropylcarbonat der Formel (V), im folgenden abgekürzt genannt: Kat. 5
Man verfuhr analog den Angaben zur Herstellung von Kat. 4, verwendete jedoch als Ausgangsstoffe 51,5 Gew.-Teile (0,5 Mol) N,N-Dimethylaminopropanol und 90,1 Gew.-Teile (1 Mol) Dimethylcarbonat.

| Elementaranalyse (C₇H₁₅NO₃) | | | | |
|---|---|---|---|---|
| berechnet [Gew.-%] | C 52,2; | H 9,4; | N 8,7; | O 29,8 |
| gefunden [Gew.-%] | C 51,6; | H 9,4; | N 8,9; | O 30,7 |

Zersetzungsbereich: 200 bis 220°C
6. Herstellung von N,N,N-Trimethylammoniumhexylcarbonat der Formel (VI), im folgenden abgekürzt genannt: Kat. 6
Man verfuhr analog den Angaben zur Herstellung von Kat. 4, verwendete jedoch als Ausgangsstoffe 72,6 Gew.-Teile (0,5 Mol) N,N-Dimethylaminohexanol-1 und 90,1 Gew.-Teile (1 Mol) Dimethylcarbonat.

| Elementaranalyse (C₁₀H₂₁NO₃) | | | | |
|---|---|---|---|---|
| berechnet [Gew.-%] | C 59,1; | H 10,3; | N 6,9; | O 23,7 |
| gefunden [Gew.-%] | C 58,5; | H 10,3; | N 7,1; | O 24,2 |

Zersetzungsbereich: 240 bis 260°C

### Herstellung der Isocyanuratgruppen aufweisenden Polyisocyanaten

### Beispiele 1 bis 19 und Vergleichsbeispiele I bis III

Die (cyclo)aliphatischen Diisocyanate wurden unter Rühren in einer Inertgasatmosphäre, z.B. Stickstoff oder Argon, auf eine Reaktionstemperatur im Bereich von 30 bis 110°C erwärmt, eine Katalysatorlösung hinzugefügt und in einem Zeitraum von 60 bis 120 Minuten trimerisiert. Zur Beendigung der Trimerisierungsreaktion wurde der Katalysator durch Zugabe von Dibutylphosphat desaktiviert.

Als Katalysator wurde ferner N-(2-Hydroxypropyl)-N,N,N-trimethylammonium-2-ethylhexanoat, im folgenden abgekürzt Kat. 0 genannt, als Vergleichssubstanz verwendet.

Die verwendeten (cyclo)aliphatischen Diisocyanate, die Katalysatorart, -menge und -lösung, die Reaktionstemperatur und -zeit und der Isocyanatgehalt und die Farbzahl der hergestellten Isocyanuratgruppen aufweisenden Polyisocyanate sind in der folgenden Tabelle zusammengefaßt.

In der Tabelle bedeuten ferner:
- HDI:: Durch thermische Spaltung vom Hexamethylen-dibutyl-urethan hergestelltes 1,6-Hexamethylen-diisocyanat
- IPDI:: 1-Isocyanato-3-isocyanatomethyl-3,5,5-trimethylcyclohexan
- BEPDI:: 2-Butyl-2-ethyl-pentamethylen-diisocyanat-1,5

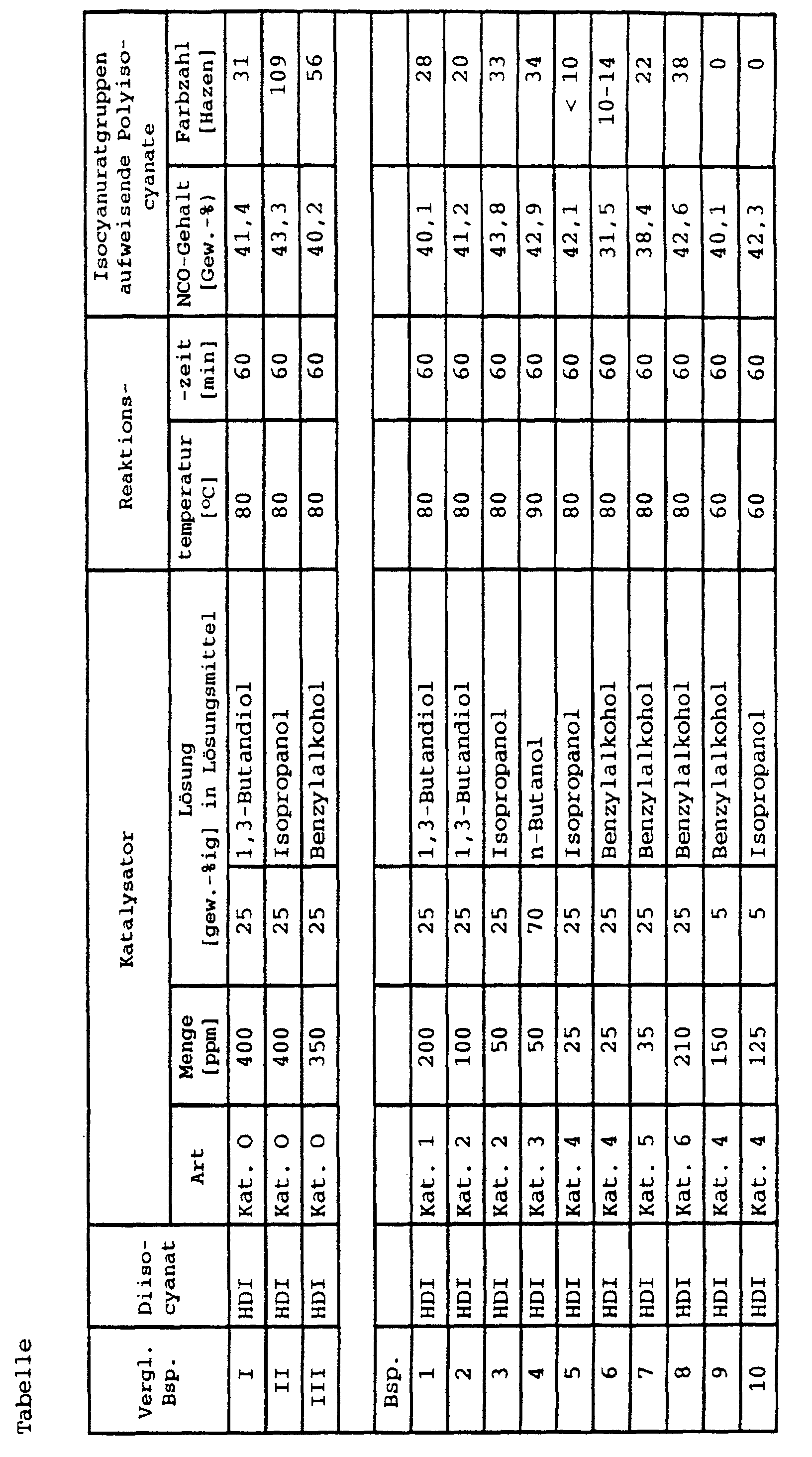

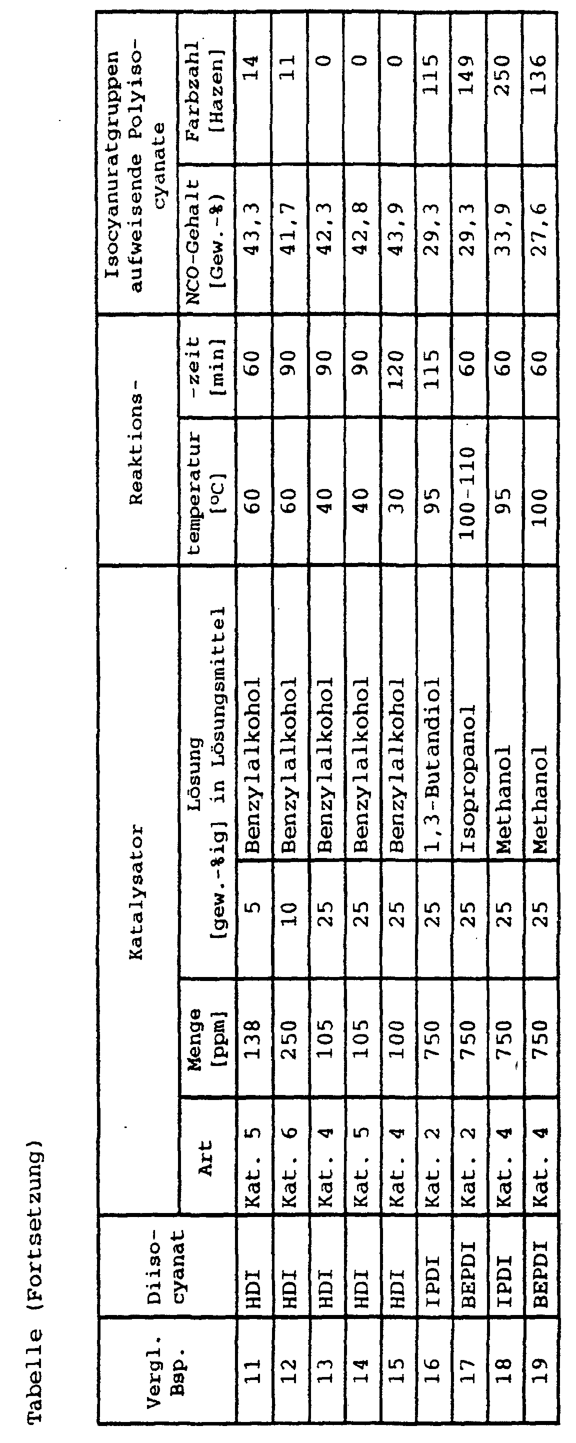

## Patentansprüche

1. Verfahren zur Herstellung von Isocyanuratgruppen aufweisenden Polyisocyanaten durch partielle Trimerisierung von aliphatischen oder/und cycloaliphatischen Diisocyanaten in Gegenwart mindestens eines Trimerisierungskatalysators und Desaktivierung des Trimerisierungskatalysators nach Erreichen des angestrebten Trimerisierungsgrads, **dadurch gekennzeichnet, daß** man als Trimerisierungskatalysatoren Tetraalkylammoniumalkylcarbonate der Formel (I) 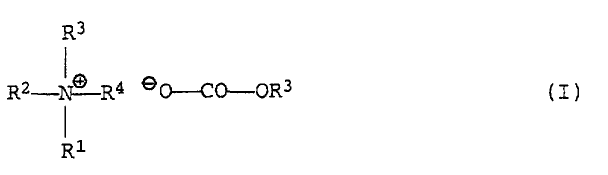 oder betainstrukturierte (Quartär-Ammonioalkyl)-carbonate der Formel (II) 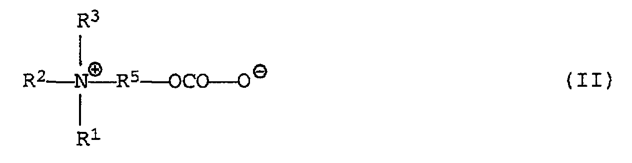 oder Mischungen aus (I) und (II) verwendet, in denen
R¹, R² und R⁴ gleich oder verschieden sind und für eine C₁-bis C₂₀-Alkylgruppe, eine C₅- bis C₆-Cycloalkylgruppe, eine C₇- bis C₁₀-Aralkylgruppe oder eine Phenylgruppe stehen oder in denen R¹ und R² zusammen mit dem Stickstoffatom einen 5- oder 6-gliedrigen Ring bilden, der noch ein zusätzliches Stickstoff- oder Sauerstoffatom als Brückenglied enthalten kann, oder in denen R¹, R² und R⁴ gemeinsam mit dem Stickstoffatom ein mehrgliedriges Mehrringsystem bilden, das noch ein oder mehrere zusätzliche Stickstoffatome und/oder Sauerstoffatome als Brückenglieder enthalten kann,
R³ eine C₁- bis C₄-Alkylgruppe ist,
R⁵ eine C₂- bis C₂₀-Alkylengruppe, eine C₅- bis C₆-Cycloalkylengruppe, eine C₇- bis C₁₀-Aralkylengruppe oder eine Phenylengruppe bedeutet oder
R⁵ und R¹ zusammen eine Alkylengruppe sind, die gemeinsam mit einem Stickstoffatom als Brückenglied einen 5- bis 7-gliedrigen heterocyclischen Ring bilden.

2. Verfahren nach Anspruch 1, **dadurch gekennzeichnet, daß** die Trimerisierungskatalysatoren ausgewählt sind aus Tetraalkylammoniumalkylcarbonaten der Formeln 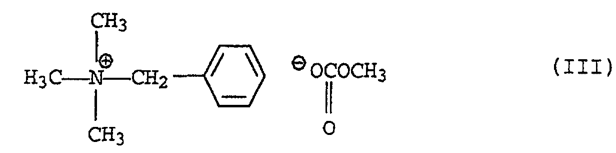 und

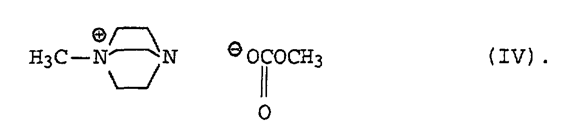

3. Verfahren nach Anspruch 1, **dadurch gekennzeichnet, daß** die Trimerisierungskatalysatoren ausgewählt sind aus betainstrukturierten (Quartär-Ammonioalkyl)-carbonaten der Formeln 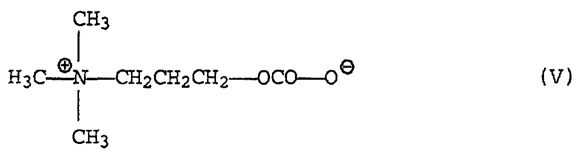 und

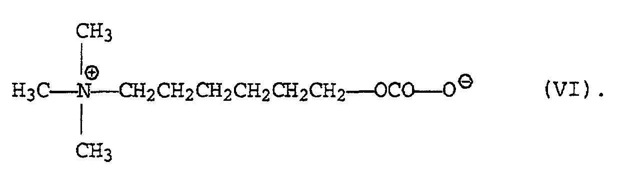

4. Verfahren nach Anspruch 1, **dadurch gekennzeichnet, daß** die partielle Trimerisierung bei einer Temperatur von 30 bis 150°C durchgeführt wird.

5. Verfahren nach Anspruch 1, **dadurch gekennzeichnet, daß** die Tetraalkylammoniumalkylcarbonate (I) oder betainstrukturierten (Quartär-Ammonioalkyl)-carbonate (II) durch Zugabe von Dibutylphosphat desaktiviert werden.

6. Verfahren nach Anspruch 1, **dadurch gekennzeichnet, daß** man nach Erreichen des angestrebten Trimerisierungsgrades und Desaktivierung des Trimerisierungskatalysators die nicht umgesetzten monomeren (cyclo)aliphatischen Diisocyanate abtrennt.

7. Verfahren nach Anspruch 1, **dadurch gekennzeichnet, daß** zur partiellen Trimerisierung nach einem phosgenfreien Verfahren hergestellte (cyclo)aliphatische Diisocyanate verwendet werden.

8. Verfahren nach Anspruch 1, **dadurch gekennzeichnet, daß** zur partiellen Trimerisierung durch thermische Spaltung von (cyclo)aliphatischen Dicarbaminsäureestern erhältliche (cyclo)aliphatische Diisocyanate verwendet werden.

9. Verfahren nach Anspruch 1, **dadurch gekennzeichnet, daß** zur partiellen Trimerisierung durch thermische Spaltung von (cyclo)aliphatischen Dicarbaminsäureestern erhältliche (cyclo)aliphatische Diisocyanate aus der Gruppe 1,6-Hexamethylen-diisocyanat, 2-Butyl-2-ethyl-pentamethylendiisocyanat-1,5 und 1-Isocyanato-3-isocyanatomethyl-3,5,5-trimethylcyclohexan verwendet werden.

10. Verfahren zur Herstellung von Isocyanatgruppen aufweisenden Polyisocyanaten durch partielle Trimerisierung von aliphatischen und/oder cycloaliphaten Diisocyanaten in Gegenwart mindestens eines Trimerisierungskatalysators und Desaktivierung des Trimerisierungskatalysators nach Erreichen des angestrebten Trimerisierungsgrades, **dadurch gekennzeichnet, daß** man als Trimerisierungskatalysator eine Verbindung der Formel (VII) 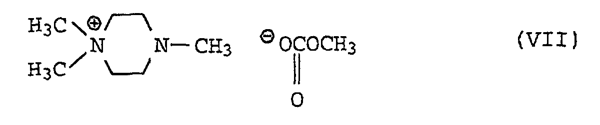 einsetzt.

11. Verfahren zur Herstellung von Isocyanatgruppen aufweisenden Polyisocyanaten durch partielle Trimerisierung von aliphatischen und/oder cycloaliphatischen Diisocyanaten in Gegenwart mindestens eines Trimerisierungskatalysators und Desaktivierung des Trimerisierungskatalysators nach Erreichen des angestrebten Trimerisierungsgrades, **dadurch gekennzeichnet, daß** man als Trimerisierungskatalysator eine Verbindung der Formel (VIII) 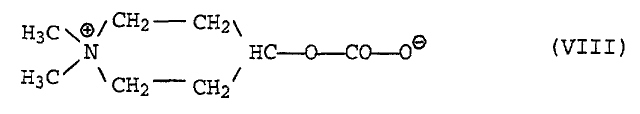 einsetzt.

## Claims

1. A process for preparing polyisocyanates containing isocyanurate groups by partial trimerization of aliphatic and/or cycloaliphatic diisocyanates in the presence of at least one trimerization catalyst and deactivation of the trimerization catalyst after reaching the desired degree of trimerization, wherein the trimerization catalysts used are tetraalkylammonium alkyl carbonates of the formula (I) 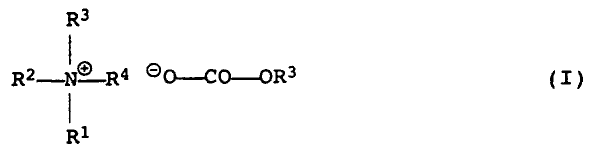 or (quaternary ammonioalkyl) carbonates having a betaine structure and the formula (II) 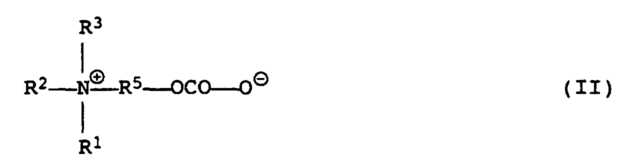 or mixtures of (I) and (II), where
R¹, R² and R⁴ are identical or different and are a C₁- to C₂₀-alkyl group, a C₅- to C₆-cycloalkyl group, a C₇- to C₁₀―aralkyl group or a phenyl group or where R¹ and R² together with the nitrogen atom form a 5-membered or 6―membered ring which can contain an additional nitrogen or oxygen atom as a bridge member, or where R¹, R² and R⁴ together with the nitrogen atom form a multi-membered multi-ring system which can contain one or more additional nitrogen atoms and/or oxygen atoms as bridge members,
R³ is a C₁- to C₄-alkyl group,
R⁵ is a C₂- to C₂₀-alkylene group, a C₅- to C₆-cycloalkylene group, a C₇- to C₁₀-aralkylene group or a phenylene group or
R⁵ and R¹ together are an alkylene group and together with a nitrogen atom as bridge member form a 5-membered to 7-membered heterocyclic ring.

2. A process as claimed in claim 1, wherein the trimerization catalysts are selected from among tetraalkylammonium alkyl carbonates of the formulae 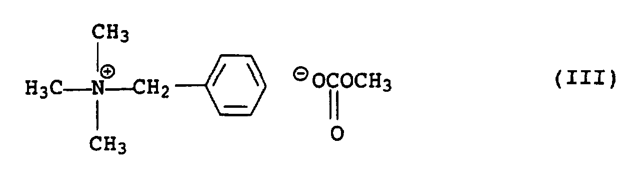 and

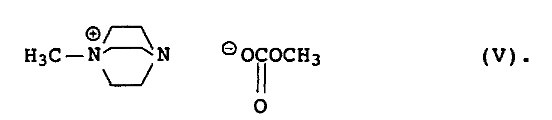

3. A process as claimed in claim 1, wherein the trimerization catalysts are selected from among (quaternary ammonioalkyl) carbonates having a betaine structure and the formulae 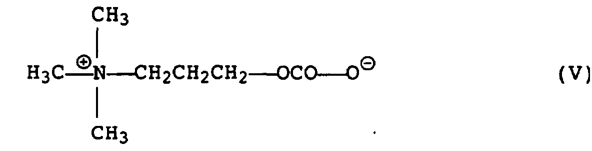 and

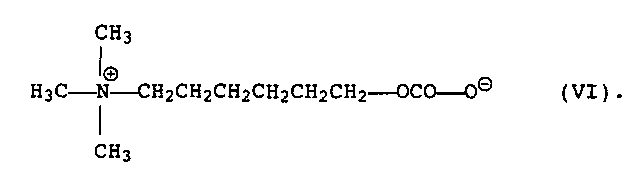

4. A process as claimed in claim 1, wherein the partial trimerization is carried out at from 30 to 150°C.

5. A process as claimed in claim 1, wherein the tetraalkylammonium alkyl carbonates (I) or (quaternary ammonioalkyl) carbonates having a betaine structure (II) are deactivated by addition of dibutyl phosphate.

6. A process as claimed in claim 1, wherein, after reaching the desired degree of trimerization and deactivating the trimerization catalyst, the unreacted monomeric (cyclo)aliphatic diisocyanates are removed.

7. A process as claimed in claim 1, wherein the partial trimerization is carried out using (cyclo)aliphatic diisocyanates prepared by a phosgene-free process.

8. A process as claimed in claim 1, wherein the partial trimerization is carried out using (cyclo)aliphatic diisocyanates obtainable by thermal cleavage of (cyclo)aliphatic dicarbamic esters.

9. A process as claimed in claim 1, wherein the partial trimerization is carried out using (cyclo)aliphatic diisocyanates obtainable by thermal cleavage of (cyclo)aliphatic dicarbamic esters and selected from the group consisting of 1,6-hexamethylenediisocyanate, 2-butyl-2-ethylpentamethylene-1,5-diisocyanate and 1-isocyanato-3-isocyanatomethyl-3,5,5-trimethylcyclohexane.

10. A process for preparing polyisocyanates containing isocyanate groups by partial trimerization of aliphatic and/or cycloaliphatic diisocyanates in the presence of at least one trimerization catalyst and deactivation of the trimerization catalyst after reaching the desired degree of trimerization, wherein the trimerization catalyst used is a compound of the formula (VII)

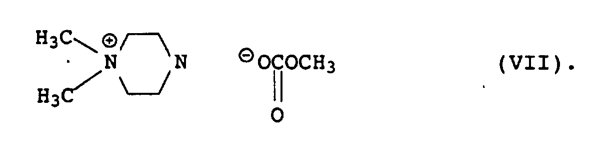

11. A process for preparing polyisocyanates containing isocyanate groups by partial trimerization of aliphatic and/or cycloaliphatic diisocyanates in the presence of at least one trimerization catalyst and deactivation of the trimerization catalyst after reaching the desired degree of trimerization, wherein the trimerization catalyst used is a compound of the formula (VIII)

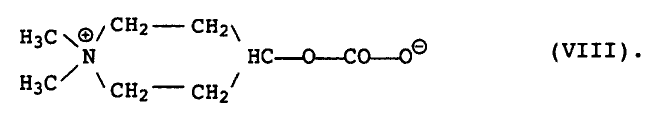

## Revendications

1. Procédé de préparation de polyisocyanates contenant des groupes isocyanurate par trimérisation partielle de diisocyanates aliphatiques ou/et cycloaliphatiques en présence d'au moins un catalyseur de trimérisation, et désactivation du catalyseur de trimérisation une fois atteint le degré de trimérisation visé, **caractérisé en ce que** l'on utilise comme catalyseurs de trimérisation des carbonates de tétraalkylammoniumalkyle de la formule (I) 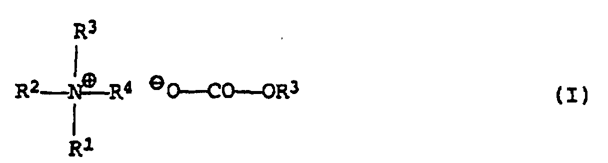 ou des carbonates d'ammonioalkyle quaternaire à structure de bétaïne de la formule (II) 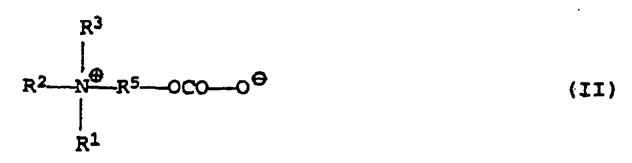 ou des mélanges de (I) et (II), dans lesquels
R¹, R² et R⁴ sont identiques ou différents et représentent un radical alkyle en C₁ à C₂₀, un radical cycloalkyle en C₅ à C₆, un radical aralkyle en C₇ à C₁₀ ou un radical phényle, ou bien où R¹ et R² forment ensemble avec l'atome d'azote un noyau pentagonal à hexagonal, qui peut encore contenir un atome supplémentaire d'azote ou d'oxygène en tant qu'élément de pontage, ou bien où R¹, R² et R⁴ forment ensemble avec l'atome d'azote un système polynucléaire à plusieurs membres, qui peut encore contenir un ou plusieurs atomes d'azote et/ou atomes d'oxygène additionnels en tant qu'éléments de pontage,
R³ représente un radical alkyle en C₁ à C₄,
R⁵ représente un radical alkylène en C₂ à C₂₀, un radical cycloalkylène en C₅ à C₆, un radical aralkylène en C₇ à C₁₀ ou un radical phénylène, ou bien
R⁵ et R¹ représentent ensemble un radical alkylène formant ensemble avec un atome d'azote en tant qu'élément de pontage un noyau hétérocyclique pentagonal à heptagonal.

2. Procédé selon la revendication 1, **caractérisé en ce** les catalyseurs de trimérisation sont choisis parmi des carbonates de tétraalkylammoniumalkyle des formules 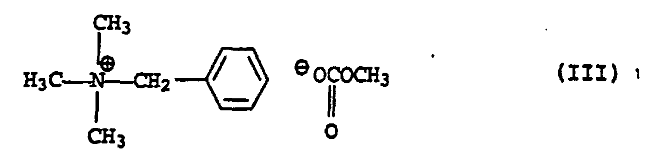 et

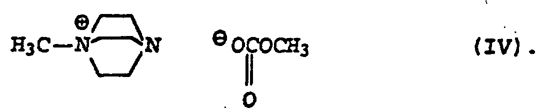

3. Procédé selon la revendication 1, **caractérisé en ce que** les catalyseurs de trimérisation sont choisis parmi des carbonates d'ammonioalkyle quaternaire à structure de bétaïne des formules 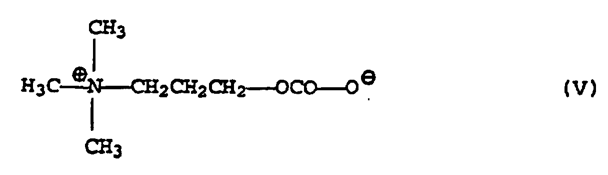 et

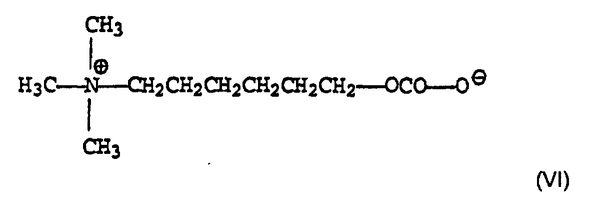

4. Procédé selon la revendication 1, **caractérisé en ce que** la trimérisation partielle est entreprise à une température de 30 à 150°C.

5. Procédé selon la revendication 1, **caractérisé en ce que** les carbonates de tétraalkylammoniumalkyle (I) ou les carbonates d'ammonioalkyle quaternaire à structure de bétaïne (II) sont désactivés par addition de phosphate de dibutyle.

6. Procédé selon la revendication 1, **caractérisé en ce qu'**une fois atteint le degré de trimérisation visé et désactivation du catalyseur de trimérisation, on sépare les diisocyanates (cyclo)aliphatiques monomères non convertis.

7. Procédé selon la revendication 1, **caractérisé en ce que** l'on utilise pour la trimérisation partielle des diisocyanates (cyclo)aliphatiques préparés par un procédé sans phosgène.

8. Procédé selon la revendication 1, **caractérisé en ce que** l'on utilise pour la trimérisation partielle des diisocyanates (cyclo)aliphatiques que l'on peut obtenir par craquage thermique d'esters (cyclo)-aliphatiques d'acide dicarbamique.

9. Procédé selon la revendication 1, **caractérisé en ce que** l'on utilise pour la trimérisation partielle des diisocyanates (cyclo)aliphatiques, que l'on peut obtenir par craquage thermique d'esters (cyclo)aliphatiques d'acide dicarbamique, qui sont choisis dans le groupe formé par le diisocyanate-1,5 de 1,6-hexaméthylène, le diisocyanate de 2-butyl-2-éthyl-pentaméthylène et le 1-isocyanato-3-isocyanatométhyl3,5,5-triméthylcyclohexane.

10. Procédé de préparation de polyisocyanates contenant des groupes isocyanate par trimérisation partielle de diisocyanates aliphatiques et/ou cycloaliphatiques en présence d'au moins un catalyseur de trimérisation, et désactivation du catalyseur de trimérisation une fois atteint le degré de trimérisation visé, **caractérisé en ce que** l'on met en oeuvre, comme catalyseur de trimérisation, un composé de la formule (VII)

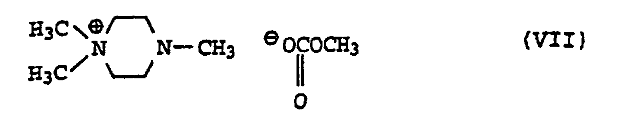

11. Procédé de préparation de polyisocyanates contenant des groupes isocyanate par trimérisation partielle de diisocyanates aliphatiques et/ou cycloaliphatiques en présence d'au moins un catalyseur de trimérisation, et désactivation du catalyseur de trimérisation une fois atteint le degré de trimérisation visé, **caractérisé en ce que** l'on met en oeuvre, comme catalyseur de trimérisation, un composé de la formule (VIII)
